# DEMANDE DE BREVET EUROPEEN

(11) **EP 1 374 798 A2**
(43) Date de publication de la demande: **02.01.2004**
(21) Numéro de dépôt: 03291469.9
(22) Date de dépôt: 17.06.2003
(51) Int. Cl.: A61F 2/01

(54) **Dispositif médical pour filtrer un liquide corporel**

(30) Priorité: 20.06.2002 FR 0207637
(71) Demandeur: B. Braun Medical SAS, 92100 Boulogne Billancourt (FR)
(72) Inventeur: Chevillon, Gérard, 92120 Montrouge (FR)
(74) Mandataire: Arnaud, Jean Pierre Alfred

(57) **Abrégé**

Un dispositif médical pour filtrer un liquide corporel présente un axe (25) et comprend une tête (21) située sensiblement selon cet axe et à laquelle sont liés des premiers éléments allongés (27a) mobiles radialement entre une position radialement déployée et une position radialement resserrée dans laquelle ils sont rapprochés de cet axe. Des seconds éléments allongés (27b) sont fixés aux premiers éléments allongés à des emplacements qui sont situés à l'écart de la tête du dispositif et d'où les seconds éléments allongés s'étendent pour définir, avec les premiers éléments allongés et la tête du dispositif, un panier de filtration pour le liquide corporel.

## Description

L'invention concerne un dispositif médical pour filtrer un liquide corporel, le dispositif présentant un axe et comprenant une tête située sensiblement selon cet axe et à laquelle sont liés des premiers éléments allongés mobiles radialement entre une position radialement déployée et une position radialement resserrée, dans laquelle ils sont rapprochés de cet axe.

Les filtres sanguins disposés dans un vaisseau de façon provisoire (filtres dits temporaires, ou filtres retirables) ou définitive (filtres définitifs) sont en particulier concernés.

Mais le dispositif de l'invention peut également potentiellement être introduit dans d'autres conduits corporels, tels que la vessie, même si tel n'est pas a priori sa destination privilégiée.

Quoi qu'il en soit, dans le domaine général des dispositifs précités que concerne l'invention, le problème à résoudre est d'abord de trouver un équilibre entre un faible encombrement radial, créé par la tête et les éléments allongés constituant les pattes mobiles du dispositif, et l'efficacité de la filtration sans occlusion.

Plus précisément, il est nécessaire d'une part de disposer d'un maillage suffisamment fin et régulier pour retenir les éléments étrangers, tels que des caillots de dimension supérieure à une valeur déterminée, et d'autre part de réduire l'encombrement du filtre qui ne doit pas constituer lui-même un obstacle trop important.

On connaît déjà, d'après le document EP-1 103 233, un filtre sanguin monobloc ayant des éléments allongés dont certains portent des crochets destinés à limiter le déplacements d'autres éléments allongés lors du passage d'un état resserré à un état dilaté.

Le document WO-00/56 245 décrit un filtre ayant des seconds éléments allongés placés très près de l'extrémité libre de premiers éléments allongés afin qu'ils augmentent le nombre d'extrémités de fils au contact de la paroi d'un vaisseau, mais sans réduction notable des ouvertures entre les éléments.

Le document WO-02/11 812 décrit un filtre n'ayant que des premiers éléments de filtre, mais comprenant en outre des organes n'ayant pas une fonction de filtre, mais seulement de support télescopique permettant d'allonger la période d'utilisation d'un filtre temporaire.

Le document EP-430 848 concerne un filtre en tulipe dans lequel des seconds éléments qui se referment en boucle à distance des extrémités des premiers éléments augmentent le maillage du côté de la tête du filtre, mais pas du côté des extrémités des premiers éléments. Ces filtres posent parfois un problème d'enroulement des pattes adjacentes dû au fait que des boucles s'emmêlent.

On pourrait envisager, pour augmenter la finesse de maillage, d'ajouter un grand nombre d'éléments allongés pour réduire la dimension des espaces les plus grands, c'est-à-dire aux extrémités des éléments. On obtiendrait cependant un filtre très occlusif augmentant le risque de thrombose.

L'invention concerne une solution équilibrée qui permet une augmentation de la finesse du maillage, mais sans accroître pratiquement les risques d'occlusion.

Selon l'invention, ce résultat est obtenu parce que des seconds éléments allongés, qui ont une extrémité libre disposée du côté opposé à une tête du filtre, sont fixés à des premiers éléments allongés partant de la tête à des emplacements situés à l'écart de la tête et qui sont plus proches de la tête du dispositif que de l'extrémité libre des premiers éléments allongés.

Plus précisément, l'invention concerne un dispositif médical pour filtrer un liquide corporel, le dispositif présentant un axe et comprenant une tête située sensiblement sur cet axe et à laquelle sont liés des premiers éléments allongés présentant au moins une extrémité libre située à l'écart de la tête du dispositif, et mobiles radialement entre une position radialement déployée et une position radialement resserrée dans laquelle ils sont rapprochés de cet axe, et des seconds éléments allongés ayant une extrémité libre disposée du côté opposé à la tête et fixés aux premiers éléments allongés à des emplacements qui sont situés à l'écart de la tête du dispositif et d'où les seconds éléments allongés s'étendent pour définir, avec les premiers éléments allongés et la tête du dispositif, un panier de filtration pour le liquide corporel ; selon l'invention, chaque second élément allongé est fixé à un premier élément allongé à un emplacement distant de la tête tel que, en position déployée, l'emplacement de fixation est à une distance de la tête qui est inférieure ou égale à sa distance à l'extrémité libre du premier élément allongé associé.

Dans un mode de réalisation particulier, les seconds éléments allongés présentent deux extrémités libres opposées et sont fixés aux premiers éléments allongés à un emplacement intermédiaire situé entre les deux extrémités libres des seconds éléments allongés.

Selon deux variantes, le nombre de seconds éléments allongés est égal à celui des premiers éléments allongés ou au double de celui des premiers éléments allongés.

Dans un mode de réalisation constituant un filtre de type permanent, le dispositif comporte des barrettes de centrage destinées à être en appui contre la paroi d'un vaisseau. De préférence, ces barrettes ont une forme de fourche dont les extrémités de deux branches sont fixées à l'extrémité libre de deux éléments allongés. Il est alors avantageux que l'extrémité des barrettes de centrage opposée aux extrémités fixées à l'extrémité libre de deux éléments allongés se trouve, dans la position déployée du filtre, à proximité de la tête du filtre en direction parallèle à l'axe. Comme ce mode de réalisation constitue un filtre de type permanent, il est avantageux qu'il comporte en outre des crochets tournés vers l'extérieur du filtre lorsque celui-ci est en position déployée.

Les avantages de ces dispositifs sont notamment les suivants :
- les ramifications créées par les seconds éléments allongés augmentent la finesse du maillage, et donc diminuent la perméabilité du filtre vis-à-vis des produits à retenir,
- l'encombrement de la tête reste limité : seuls les premiers éléments y sont directement fixés, et le dispositif peut donc présenter un encombrement radial limité en position radialement resserrée des pattes, et
- l'adjonction de ces seconds éléments allongés à la périphérie du dispositif, en partie intermédiaire des premiers éléments allongés, avec une orientation qui peut être différente de celle de ces premiers éléments, permet de réaliser des formes de filtre très performantes.

La construction du filtre selon l'invention permet une miniaturisation, et donc une réduction de l'encombrement radial des dispositifs filtrants qui facilite leur introduction.

Deux modes de réalisation de l'invention sont notamment les suivants :
- les seconds éléments allongés présentent au moins une extrémité libre située à l'écart de la tête et à l'écart des emplacements de fixation des seconds éléments allongés aux premiers éléments allongés, et/ou
- les seconds éléments allongés présentent deux extrémités libres opposées et sont fixés aux premiers éléments allongés à un emplacement intermédiaire situé entre les deux extrémités libres des seconds éléments allongés.

Les seconds éléments allongés peuvent favoriser le centrage du filtre dans son conduit corporel, à la manière des barrettes ou appendices de centrage décrits dans le document FR-A-2 573 646, et participent à la filtration dans ce conduit pendant sa période d'implantation. Surtout, ces seconds éléments allongés accroissent les possibilités de réalisation du maillage filtrant.

Ainsi, l'invention concerne un filtre sanguin qui est destiné à être disposé à l'intérieur d'un vaisseau sanguin par l'intermédiaire d'une gaine flexible dans laquelle le dispositif coulisse dans son état radialement resserré. Il peut être introduit par des méthodes déjà maîtrisées dans la technique actuelle, par voie endoluminale (en particulier, la technique dite de Seldinger).

D'autres caractéristiques et avantages de l'invention seront mieux compris à la lecture de la description qui va suivre d'exemples de réalisation, faite en référence aux dessins annexés sur lesquels :
- la figure 1 représente un premier mode de réalisation d'un dispositif filtrant selon l'invention représenté dans son état radialement dilaté,
- la figure 2 représente un second mode de réalisation,
- la figure 3 représente un troisième mode de réalisation,
- la figure 4 représente un filtre sanguin dans sa gaine introductrice, et
- la figure 5 représente une variante du filtre sanguin de la figure 1.

On décrit dans la suite des dispositifs médicaux adaptés en particulier pour être introduits dans un vaisseau sanguin.

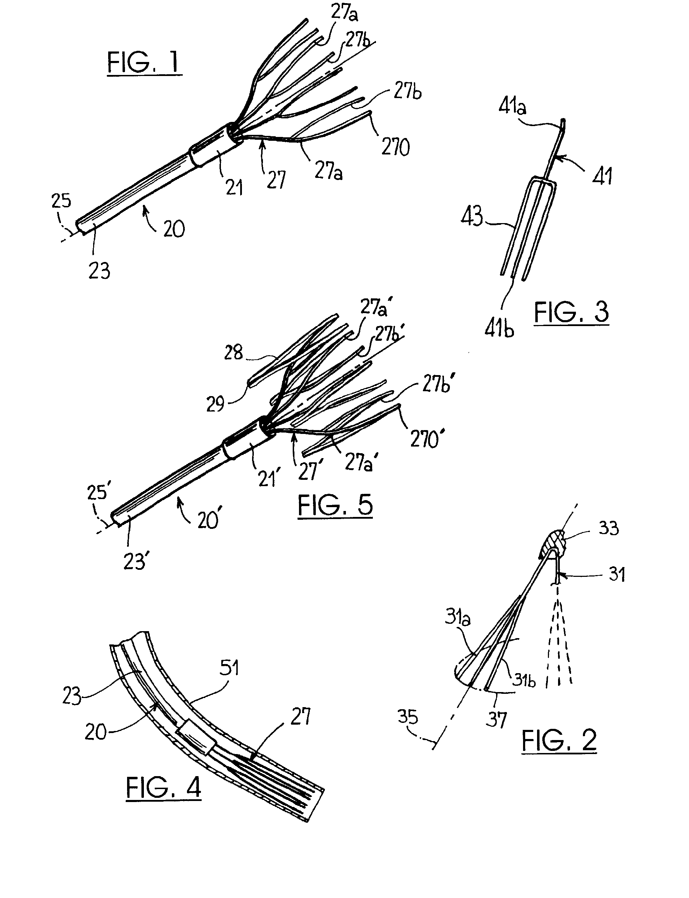

La figure 1 représente un filtre sanguin temporaire 20 possédant une tête métallique 21 reliée à un organe flexible de manoeuvre (ou tige poussoir) 23.

L'organe 23 est allongé suivant l'axe 25 du filtre et présente axialement une longueur suffisante pour que l'on puisse manoeuvrer la tête et les pattes 27 du filtre à l'endroit de sa zone d'implantation (vaisseau sanguin) depuis l'extérieur du corps du patient, par tenue à la main de l'extrémité proximale de cet organe qui peut se présenter comme un cathéter et qui a été introduit par voie endoluminale.

Sur la figure 1, les pattes 27 sont dans leur état radialement déployé et définissent une corolle tronconique. Elles comprennent chacune un premier brin (ou premier élément allongé) 27a fixé à la tête 21 (de préférence, à l'intérieur de cette tête) et relié, à distance de la tête, en partie intermédiaire entre la tête et l'extrémité libre 270, à au moins un second brin (ou second élément allongé) 27b fixé à une première extrémité, par exemple par soudage, et qui se termine, comme chaque élément 27a, par une extrémité libre.

Le filtre peut être déplacé dans le vaisseau, et il est destiné à être retiré après quelques semaines ou quelques mois d'implantation.

Les brins 27a, 27b, sont incurvés naturellement. D'autres formes sont cependant possibles. Leur matière peut être métallique (acier inoxydable ou "Nitinol" par exemple).

La figure 2 représente un premier élément allongé ou brin 31 de filtre, recourbé en "U" en partie intermédiaire de sa longueur, pour être fixé (par exemple par soudage) à l'intérieur d'une tête creuse 33 de ce filtre, de telle sorte que cet élément présente deux extrémités libres opposées. A proximité de la tête 33 sont fixés deux brins rapportés 31a, 31b, s'étendant de biais par rapport à l'axe 35 suivant lequel s'étend l'élément 31, en définissant un évasement vers l'extrémité de plus grande section du filtre indiquée par la portion de cercle 37 sur laquelle se situe également une extrémité libre du brin 31. Une réalisation symétrique, indiquée en traits interrompus, peut être incorporée. Ainsi, les pattes du filtre paraissent "ramifiées", les ramifications étant constituées par les seconds éléments (ou brins) rapportés sur les premiers qui, eux, sont directement fixés à la tête du filtre.

La figure 3 représente une forme qui n'est pas évasée à l'endroit des "ramifications" des pattes, lesquelles présentent ici un aspect en trident. Le premier élément allongé ou brin 41, fixé à l'intérieur de la tête du filtre (non représentée), à son extrémité proximale 41a, s'étend linéairement jusqu'à une extrémité distale 41b destinée à demeurer libre.

En partie intermédiaire, entre ces extrémités 41a, 41b, un second élément allongé 43 en "U renversé" est soudé au premier élément allongé 41 pour définir la forme illustrée. Bien entendu, dans une variante de réalisation, au moins deux seconds éléments allongés, chacun en L, peuvent être fixés au brin 41, avec maintien de l'extrémité distale libre, tant du premier élément allongé que des seconds éléments allongés.

La figure 4 représente le filtre temporaire du mode de réalisation de la figure 1 introduit dans une gaine souple 51 destinée à être mise en place par voie endoluminale, jusqu'à une zone d'implantation vasculaire concernée.

A l'intérieur de la gaine 51, le dispositif filtrant 20 s'étend suivant l'axe de la gaine, avec ses pattes 27 radialement resserrées.

Lorsqu'un tel filtre temporaire, avec son organe de manoeuvre 23, doit être remplacé par un filtre définitif, ou un filtre temporaire différent (en particulier sans organe de manoeuvre 23, mais avec une capacité de séparation structurelle entre le filtre et ses moyens de fixation au vaisseau, par exemple par rupture mécanique locale de pattes à proximité de crochets de fixation), une tige poussoir indépendante du filtre et destinée à coulisser à l'intérieur de la gaine 51 peut avantageusement remplacer l'organe de manoeuvre 23.

En résumé, la mise en place du dispositif filtrant de la figure 1 à l'intérieur d'un vaisseau sanguin peut s'opérer par voie haute ou basse, par exemple jugulaire, brachiale, sous-clavière, fémorale ou autre. Par cette voie d'approche, par dénudation vasculaire, ou par une approche transcutanée (endoluminale), un dispositif de ponction et d'élargissement est mis en place et permet d'introduire la gaine souple 51 dans le système vasculaire. Dans un premier temps, le dispositif filtrant est placé dans son état radialement resserré à l'intérieur de la gaine. Une fois l'extrémité distale de cette gaine positionnée à l'endroit de la zone d'intervention, la gaine est tirée vers l'arrière (vers son côté proximal), tandis que le dispositif filtrant est maintenu en position fixe par l'intermédiaire de son organe de manoeuvre 23 que l'opérateur tient en main, depuis l'extérieur du corps du patient. Ce mouvement relatif entre le dispositif filtrant 20 et la gaine 51 provoque la sortie des pattes 27 hors de la gaine. Lorsqu'elles sont auto-expansibles, comme dans les exemples illustrés, ces pattes se déploient donc d'elles-mêmes radialement jusqu'à placer le dispositif dans un état de filtration, in situ.

En fin d'intervention, le dispositif filtrant est rétracté, puis retiré par des opérations inverses des précédentes.

La figure 5 représente un filtre sanguin temporaire 20' analogue au filtre 20 de la figure 1, mais de type définitif et non temporaire. Il comporte une tête métallique 21' reliée à un organe flexible de manoeuvre 23' pour sa mise en place.

L'organe 23' est allongé suivant l'axe 25' du filtre et présente axialement une longueur suffisante pour que la tête et les pattes 27' soient manoeuvrées dans la zone d'implantation (vaisseau sanguin) depuis l'extérieur du corps du patient.

Sur la figure 5, les pattes 27' sont dans leur état radialement déployé et définissent une corolle tronconique. Elles comprennent chacune un premier brin (ou premier élément allongé) 27a' fixé à la tête 21' (de préférence, à l'intérieur de cette tête) et relié, à distance de la tête, en partie intermédiaire entre la tête et l'extrémité libre 270', à au moins un second brin (ou second élément allongé) 27b' fixé à une première extrémité, par exemple par soudage, et qui se termine, comme chaque élément 27a', par une extrémité libre. Les brins 27a', 27b', sont incurvés naturellement.

Dans cette variante, des barrettes de centrage 28 sont fixées aux extrémités libres 270' des brins ou éléments allongés. Leur extrémité 29 se trouve de préférence, en direction parallèle à l'axe 25', à proximité de la tête. On peut les comparer aux barrettes de centrage qui sont décrites dans le document FR-A-2 573 646. Comme cette variante concerne un filtre permanent, celui-ci comporte de préférence, sur les barrettes et à leurs extrémités, des crochets destinés à empêcher la migration du filtre.

## Revendications

1. Dispositif médical pour filtrer un liquide corporel, le dispositif présentant un axe (25, 25', 35) et comprenant une tête (21, 21', 33) située sensiblement sur cet axe et à laquelle sont liés des premiers éléments allongés (27a, 27a', 31, 41) présentant au moins une extrémité libre située à l'écart de la tête (21, 21', 33) du dispositif et mobiles radialement entre une position radialement déployée et une position radialement resserrée dans laquelle ils sont rapprochés de cet axe, et des seconds éléments allongés (27b; 27b' ; 31a, 31b ; 43) ayant une extrémité libre disposée du côté opposé à la tête et fixés aux premiers éléments allongés (27a, 27a', 31, 41) à des emplacements qui sont situés à l'écart de la tête du dispositif et d'où les seconds éléments allongés s'étendent pour définir, avec les premiers éléments allongés et la tête du dispositif, un panier de filtration pour le liquide corporel, **caractérisé en ce que** chaque second élément allongé (27b ; 27b' ; 31a, 31b ; 43) est fixé à un premier élément allongé (27a, 27a', 31, 41) à un emplacement distant de la tête tel que, en position déployée, l'emplacement de fixation est à une distance de la tête (21, 21', 33) qui est inférieure ou égale à sa distance à l'extrémité libre du premier élément allongé associé (27a, 27a', 31, 41).

2. Dispositif selon la revendication 1, **caractérisé en ce que** les seconds éléments allongés (43) présentent deux extrémités libres opposées et sont fixés aux premiers éléments allongés (41) à un emplacement intermédiaire situé entre les deux extrémités libres des seconds éléments allongés.

3. Dispositif selon la revendication 1, **caractérisé en ce que** le nombre de seconds éléments allongés (27b, 27b') est égal à celui des premiers éléments allongés (27a, 27a').

4. Dispositif selon l'une des revendications 1 et 2, **caractérisé en ce que** le nombre de seconds éléments allongés (31a, 31b ; 43) est égal au double de celui des premiers éléments allongés (31, 41).

5. Dispositif selon l'une quelconque des revendications précédentes, constituant un filtre de type permanent, **caractérisé en ce qu'**il comporte des barrettes de centrage (28) destinées à être en appui contre la paroi d'un vaisseau.

6. Dispositif selon la revendication 5, **caractérisé en ce que** les barrettes de centrage (28) ont une forme de fourche dont les extrémités de deux branches sont fixées à l'extrémité libre (270') de deux éléments allongés.

7. Dispositif selon la revendication 6, **caractérisé en ce que** l'extrémité (29) des barrettes de centrage (28) opposée aux extrémités fixées à l'extrémité libre de deux éléments allongés se trouve, dans la position déployée du filtre, à proximité de la tête du filtre en direction parallèle à l'axe (25').

8. Dispositif selon l'une des revendications 5 à 7, **caractérisé en ce qu'**il comporte en outre des crochets tournés vers l'extérieur du filtre lorsque celui-ci est en position déployée.
